# EUROPEAN PATENT SPECIFICATION

(11) **EP 0 813 487 B1**
(45) Date of publication and mention of the grant of the patent: **18.08.1999**
(21) Application number: 96910922.2
(22) Date of filing: 20.03.1996
(51) Int. Cl.: B65D 75/58, A61F 6/00

(54) **PACKAGE FOR A CONDOM**
KONDOMVERPACKUNG
EMBALLAGE POUR PRESERVATIF

(30) Priority: 20.03.1995 SE 9501020
(43) Date of publication of application: 29.12.1997
(73) Proprietor: Kursner, Jean-Luc, 1173 Fechy (CH)
(72) Inventor: Kursner, Jean-Luc, 1173 Fechy (CH)
(74) Representative: Johansson, Lars-Erik
(86) International application number: EP9601239
(87) International publication number: WO9629262

(56) References cited:
- WO-A-92/20595
- WO-A-95/02379
- US-A- 2 391 094
- US-A- 3 349 993
- US-A- 3 986 640
- US-A- 5 427 233

## Description

### TECHNICAL FIELD

This invention relates to packages for condoms, and particularly to easy open condom packages of the type that can be conveniently opened with one hand without damaging the condom.

### BACKGROUND ART

Condoms have become a desirable way of preventing conception and avoiding the spread of diseases. There are particular problems in connection with the storage, distribution and handling of the condoms which are quite delicate.

Each condom is packed separately. Typically the product is carried by the user in pockets, handbags, wallets etc. where it could come in contact with sharp or rough objects. The condom package has therefore to be protect the condom against contact with such objects which could destroy the same. Additionally the package must be absolutely tight to be able to keep the condom sterile. The package must also be designed so that the risk for unintentional opening of the package is minimized.

On the other hand the normal situation at the time of its use requires that the package should be easy to open, preferably by means of only one hand. Additionally, the unpacking of the condom must be possible to make in a safe way so that the condom is not damaged or dropped during this operation. Further the package must offer a good security against tampering. It should preferably be possible for the consumer at the moment of its use to indicate that the condom has not been tampered with.

The document WO92/20595 shows a package for a condom having a base with edges and a cover secured to the edges of the base and covering a condom between the base and the cover. The cover is provided with weakenings to facilitate breaking of the cover when the package is opened. The weakenings could be arranged in different directions across the cover and are always reaching all the way from one edge to the opposite. Such a package will be very easy to open but the problem of protection of the condom and the risk of unintentional opening of the package have not been considered.

Furthermore said document illustrates a method of opening the package by means of the fingertips. The further description of the present invention will describe a quite different way of opening the package according to the invention. The palm of the hand is used for the opening. The package according to WO92/20595, round or square, has a diameter which is only slightly larger than the diameter of the condom. This means that it would be impossible or at least very difficult to open that package with the palm of the hand.

Many consumers are also sensitive to the fact that a condom package could be considered as embarrassing or provocative in certain situations.

To solve this last mentioned problem a type of packages for condoms has been proposed disguising its real nature. The document WO88/07650 e.g. shows a package of this kind forming part of a cigarette lighter. Another package of the same category is disclosed in the document WO88/06534. In this case the condom package forms part of a ball-point pen.

One disadvantage of this type of package where the condom is contained in an object which has a completely different use is that when using the object e.g. a cigarette lighter, the safety of the condom might be deteriorated, in this particular case by the generated heat. Another problem is that this type of common objects which can also be used for another purpose are typically objects you easily mislay.

### SUMMARY OF THE INVENTION

One object of the invention is to improve condom packages in essential respects such as safety, discreet appearance etc. The package according to the invention also introduces a new method of opening condom packages.

Thus one object of the invention is to provide a condom package which at first sight has the outer appearance of a well known object, a credit card, although it can not be used as such.

The package is designed in such a way that it can easily be opened in the grip between the thumb and one or several of the other fingers on one hand. It can also be opened in the palm of the hand.

These and other objects are attained by means of a package for a condom characterised in that the package has the general appearance and dimensions, except for the thickness, of a standardised credit card, that both the upper and lower parts (1, 2) are made of rigid plastic material, that at least one of the upper and lower parts (1, 2) is provided with at least one weakening arranged reaching essentially from the middle of one of the edges in a pair of opposite edges on said part in a direction essentially perpendicular to said edges, less than half the distance between said edges.

### BRIEF DESCRIPTION OF THE DRAWINGS

Other characteristics, objects and advantages of the invention will become evident from the following detailed description when read in light of the accompanying drawings on which
Figure 1 is a perspective view showing an embodiment of the condom package according to the invention
Figure 2a is a plane view of the package according to figure 1.
Figure 2b is a section A - A of an embodiment of the condom package shown in figure 2a.
Figure 2c is a section A - A of another embodiment of the condom package shown in figure 2a.
Figure 2d is a section of an embodiment of the condom package in which the cavity reaches the edge of the card.
Figure 3 shows a part of the package in figure 2b provided with weakenings.
Figure 4 shows schematically an arrangement on the inside of one embodiment of the package with protection foils
Figure 5 shows an opened package with the protection foils according to figure 4.
Figure 6 shows a method to open a condom package according to the invention.
Figures 7 and 8 show further examples of opened packages.

### DETAILED DESCRIPTION OF THE INVENTION

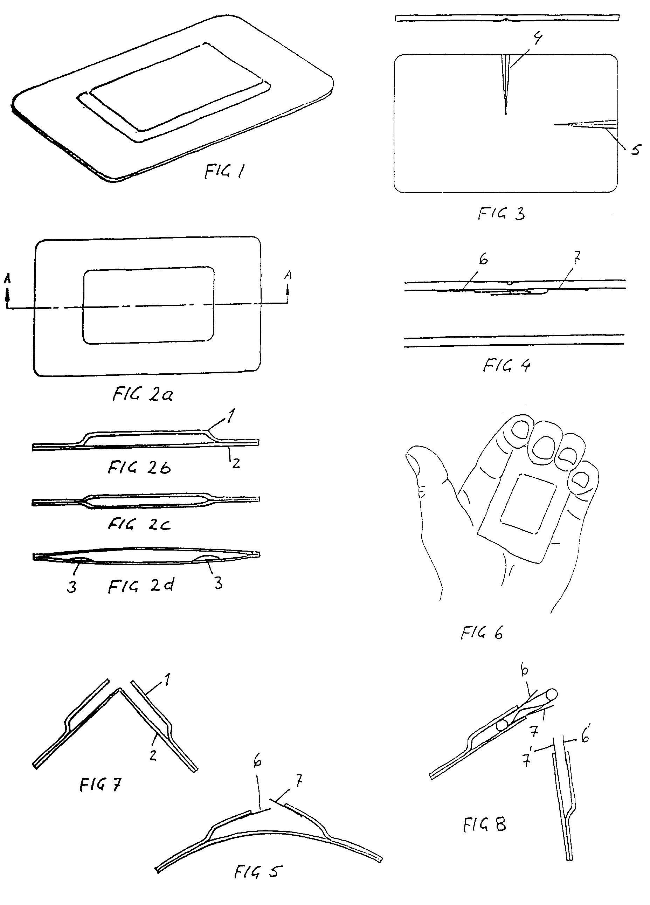

Figure 1 shows in a perspective view an embodiment of the condom package according to the invention. Except for the thickness the outer dimensions of the package are matched to the standardised dimensions of a credit card. Due to this form of the package the consumer is naturally carrying one or several packages together with ordinary plastic cards, type credit cards, telephone cards, membership cards etc. all of the same standardised dimensions. Wallets etc. are usually organised in such a way that plastic cards are arranged together. This means that the condom package will be profiting from the extra stability and protection which is provided by the neighbouring cards. Plastic cards are also usually kept under the safest possible conditions which means that the risk of mislay is minimised. The fact that the package has no additional use (pen, lighter etc.) also contributes to minimise the risk of mislay.

Additionally the appearance as an ordinary credit card is absolutely non-provocative for people sensitive on that point.

As will be described below both sides of the package are preferably made of a rigid plastic material which increases the security for the condom during storage and handling of the package. Text or graphics could preferably be added to the rather big flat surfaces of the package. A number of techniques exist for adding text or graphics on to the surface of a credit card. These techniques could be used to add instructions in relation to the actual product, i.e. the condom, or decorative elements or unrelated advertising text etc. In this respect the double sided essentially flat appearance of the package is very favorable.

The package could be made of e.g. a suitable thermoplastic material. The choice of thermoplastic materials as well as thickness, special weakenings etc. for the two sides of the package are dependent on the particular embodiment.

As will be understood by those skilled in the art, what will be described are preferred embodiments in which modifications and changes may be made without departing from the spirit and the scope of the invention.

Figure 2 shows a plane view of the package according to figure 1 and sections of a few embodiments of the package in form of a credit card.

The illustrated embodiments show a package made of two generally flat, rigid, parts sealed together along their edges forming a cavity for the condom in between. This structure makes the package rather stiff and gives good protection for the condom.

The embodiment shown in figure 2b is unsymmetrical in the plane of the card which means that the card will be more easy to bend in one direction.

The sections of figures 2c and 2d are both symmetrical. The two parts are secured together along the peripheral edge by means of e.g. a suitable adhesive or by means of the application of ultra sound technology or any other procedure known in the technical field. The cavity defined between the two parts 1 and 2 is fluid-tight in order to protect the condom.

With a comparatively rigid container which is larger than actually needed for the condom, pockets with antifriction, gliding liquid 3 could be added in the container. These pockets or separate containers could then, if so wished, be opened by means of external pressure before the actual condom package is opened. Such an arrangement would not be possible with the ordinary soft packages common today or packages in the form of lighters, pens etc..

Fig 3 illustrates a package with one side 2 weakened along two transverse directions. The weakenings 4, 5 are only extending across part of the package length and width. Other embodiments could be envisaged which have weakenings only in one direction or weakenings, or a weakening, which extends all over the length or width.

Both sides 1 and 2 of the package could be provided with weakenings if preferred. This would be most suitable in embodiments with sections according to figures 2c and 2d when the card is arranged to be opened by bending the same in either direction.

In a preferred embodiment the material of at least one of the parts 1 and 2 is rigid and substantially unstretchable. This means that the material responds to tension by yielding only slightly before breaking. Figure 7 shows a package of this type after opening.

In an embodiment as shown in figure 3 with weakenings extending from the outer periphery of the two sides towards the middle of the card, the opening could be achieved in either of two perpendicular directions. The weakenings in one of the parts 1 or 2 could for instance provoke a sharp bending of the card in a defined area which will provoke the tearing of the material in the opposite part.

The weakening as such could alternatively be the starting point for the rupture.

The material of at least one of the parts 1 and 2 is in this case of such a nature that fractures propagate readily in it.

In general it should be noted that the material in the package could be different on the two sides.

Figure 4 shows an arrangement on the inside of the cavity between the two parts 1 and 2. In order to protect the condom when taken out from the opened package two plastic foils 6, 7 have been arranged on the inside partly covering each other, overlapping the rupture zone. When the package is arranged to be opened in both directions, plastic foils have to be arranged on both sides of the cavity.

Figure 5 shows a package having these plastic foils when opened. It has turned out that the plastic foils contribute to keep the condom accessible in the opening minimising the risk to drop the same.

In this example the weakening is arranged on the side which is ruptured. The other part 1 or 2 is only bending.

In figure 7 a weakening is also, or exclusively, arranged on the part 1 or 2 which is not ruptured.

Figure 8 shows a package in which the material characteristics, weakenings etc. have been chosen in such a way that the package when bent will break in two. The plastic foils will help to keep the condom gripped by one of the two parts.

A person using the package can open the package simply by pressing it between the thumb and one or several of the other fingers of one hand. Alternatively, the user may open the package in the way illustrated in figure 6. The card is then preferably bent in the direction of the palm of the hand. Of course in all cases two hands may be used if convenient.

The materials, thickness and other characteristics of the two sides of the card are such that the pressure from the palm of the hand is sufficient to bend the card and break open one side of the package.

As understood from the above the dimensions of the package, the material and the design, cf. especially figures 2b, 5, 7 and 8, makes it possible using the technique illustrated in figure 6 to open the package in the direction into the palm of the hand which minimises the risk of dropping the condom.

According to yet another embodiment of the invention the packages shown have the air flushed out with nitrogen or other neutral gases to protect the condoms from oxygen degradation.

When the package is opened a certain sound will be which constitutes a safety indication that the package has not been subject to tampering.

The condom could also be vacuum packed. Sudden entry of gases upon the cover rupturing also creates a sound providing evidence that the package has not been tampered with.

In the embodiment according to figure 7 the design of the package makes it especially easy to remove the condom with the same hand as has been used for the opening.

As should be evident from the above description of various embodiments, without a considerable bending of the card the risk for opening is minimal.

## Claims

1. A package for condoms comprising an upper (1) and a lower (2) generally flat part sealed to each other along their edges, defining a cavity for the condom therebetween, **characterised in that** the package has the general appearance and dimensions, except for the thickness, of a standardised credit card, that both the upper and lower parts (1, 2) are made of rigid material, that at least one of the upper and lower parts (1, 2) is provided with at least one weakening arranged reaching essentially from the middle of one of the edges in a pair of opposite edges on said part in a direction essentially perpendicular to said edges, less than half the distance between said edges.

2. A package according to claim 1, **characterised in that** one of the upper and lower parts (1, 2) is provided with two essentially perpendicular weakenings reaching in the direction of the center of said part from two perpendicular edges of said part.

3. A package according to claim 1, **characterised in that** at least one the parts (1, 2) on the cavity side is provided with a protection foil overlapping the rupture zone.

4. A package according to claim 3, **characterised in that** said part (1, 2 is provided with two protection foils overlapping each other and the rupture zone.

5. A package according to claim 1, **characterised in that** one of the parts (2) is generally flat and the other (1) is provided with the cavity forming shape making the section perpendicular to the plane of the package unsymmetrical and the package more easy to bend for opening in one direction.

6. A package according to one of the claims 1 to 5, **characterised in that** a pocket containing a lubricant is arranged inside the cavity arranged to be opened before the opening of the package by means of forces applied to the opposite sides of the package.

## Patentansprüche

1. Kondomverpackung, umfassend einen oberen (1) und einen unteren (2) im wesentlichen flachen Teil, die entlang ihrer Kanten dichtend miteinander verbunden sind, was dazwischen einen Hohlraum für das Kondom definiert, dadurch gekennzeichnet, daß die Verpackung das allgemeine Erscheinungsbild und mit Ausnahme der Dicke die Abmessungen einer standardisierten Kreditkarte aufweist, daß sowohl der obere als auch der untere Teil (1, 2) aus starrem Werkstoff hergestellt sind, daß der obere und/oder untere Teil (1, 2) mit zumindest einer Schwächung versehen sind/ist, die im wesentlichen von der Mitte einer der Kanten eines Paars gegenüberliegender Kanten an dem Teil in eine Richtung im wesentlichen rechtwinklig zu den Kanten über weniger als die Hälfte der Entfernung zwischen den Kanten reichend angeordnet ist.

2. Verpackung nach Anspruch 1, dadurch gekennzeichnet, daß der obere oder der untere Teil (1, 2) mit zwei im wesentlichen rechtwinkligen Schwächungen versehen ist, die von zwei rechtwinkligen Kanten des Teils zur Richtung der Mitte des Teils reichen.

3. Verpackung nach Anspruch 1, dadurch gekennzeichnet, daß zumindest einer der Teile (1, 2) auf der Hohlraumeite mit einer die Rißzone überlappenden Schutzfolie versehen ist.

4. Verpackung nach Anspruch 3, dadurch gekennzeichnet, daß der Teil (1, 2) mit zwei Schutzfolien versehen ist, die einander und die Rißzone überlappen.

5. Verpackung nach Anspruch 1, dadurch gekennzeichnet, daß einer der Teile (2) im wesentlichen flach und der andere (1) mit der hohlraumbildenden Form versehen ist, was das Profil rechtwinklig zur Ebene der Verpackung unsymmetrisch und die Verpackung zum Öffnen in einer Richtung leichter biegbar macht.

6. Verpackung nach einem der Ansprüche 1 bis 5, dadurch gekennzeichnet, daß innerhalb des Hohlraums eine ein Gleitmittel enthaltende Tasche angeordnet ist, so daß sie vor dem Öffnen der Verpackung mittels auf die gegenüberliegenden Seiten der Verpackung aufgebrachten Kräften zu öffnen ist.

## Revendications

1. Emballage pour préservatif, comprenant une partie supérieure (1) et une partie inférieure (2) généralement plates, scellées l'une à l'autre le long de leurs bords, pour définir entre elles une cavité destinée à recevoir le préservatif,
caractérisé en ce que
• l'emballage présente l'aspect général et les dimensions, sauf pour l'épaisseur, d'une carte de crédit standard,
• la partie supérieure (1) et la partie inférieure (2) sont toutes deux réalisées dans un matériau rigide, et
• l'une au moins de la partie supérieure (1) et de la partie inférieure (2) est munie d'au moins un affaiblissement disposé pour s'étendre essentiellement, à partir du milieu de l'un des bords, en une paire de bords opposés sur cette partie, dans une direction essentiellement perpendiculaire aux bords, sur moins de la moitié de la distance entre les bords.

2. Emballage selon la revendication 1,
caractérisé en ce que
l'une de la partie supérieure (1) et de la partie inférieure (2) est munie de deux affaiblissements essentiellement perpendiculaires s'étendant dans la direction dirigée vers le centre de cette partie, en partant des deux bords perpendiculaires de celle-ci.

3. Emballage selon la revendication 1,
caractérisé en ce que
l'une au moins des parties (1, 2) du côté de la cavité, est munie d'une feuille de protection recouvrant la zone de rupture.

4. Emballage selon la revendication 3,
caractérisé en ce que
la partie (1, 2) est munie de deux feuilles de protection se recouvrant l'une l'autre et recouvrant la zone de rupture.

5. Emballage selon la revendication 1,
caractérisé en ce que
l'une (2) des parties est généralement plate et l'autre (1) est munie de la forme de cavité rendant dissymétrique la section perpendiculaire au plan de l'emballage, et rendant l'emballage plus facile à courber pour l'ouvrir dans une direction.

6. Emballage selon l'une des revendications 1 à 5,
caractérisé en ce qu'
une poche contenant un lubrifiant est disposée à l'intérieur de la cavité de manière à s'ouvrir avant l'ouverture de l'emballage, sous l'action de forces appliquées aux côtés opposés de l'emballage.
